(19) Europäisches Patentamt

European Patent Office

Office européen des brevets

(11) **EP 3 654 248 A1**

(12) **EUROPEAN PATENT APPLICATION**

(43) Date of publication:
**20.05.2020  Bulletin 2020/21**

(51) Int Cl.:
**G06N 3/04** (2006.01)          **G06N 3/08** (2006.01)

(21) Application number: **18206946.8**

(22) Date of filing: **19.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Siemens Aktiengesellschaft 80333 München (DE)**

(72) Inventor: **Gu, Jindong 81375 München (DE)**

(54)  **VERIFICATION OF CLASSIFICATION DECISIONS IN CONVOLUTIONAL NEURAL NETWORKS**

(57)      In one aspect the invention relates to a computer-implemented method for providing a computer-implemented method for verifying a visual classification architecture of a convolutional neural network (CNN) and its decisions The method comprises to access (S1) a memory (MEM) with a convolutional neural network (CNN), being trained for a visual classification task into a set of target classes (tc); to use (S2) the convolutional neural network (CNN) for an input image (12) and after a forward pass of the convolutional neural network (CNN), in a backward pass: to apply (S3) a contrastive layer-wise relevance propagation algorithm (CLRP) or to apply (S4) a Bottom Up Attention pattern (BUAP), which is implicitly learned by the convolutional neural network (CNN) for providing (S5) a verification signal (vs).

# FIG 4

EP 3 654 248 A1

**Description**

Convolutional Neural Networks (in the following abbreviated as CNN) have achieved great success in different technical application fields, like medical imaging and computer vision in general in recent years. Benefiting from large-scale training data, (e.g. ImageNet), CNNs are capable of learning filters and image compositions at the same time. Various approaches have been adopted to further increase generalization ability of CNNs. CNNs may for example be applied for classification tasks in several technical fields, like medical imaging (distinguishing e.g. healthy image parts from lesions) or in production (e.g. classifying products in waste or not).

However, if a trained CNN is used, the classification result may not be subject to a step by step verification throughout the network architecture. Thus, the internal working of the CNN is "hidden", such as the final decision of the CNN is not retraceable for each neuron in the network and thus not known. The provided result is to be trusted. However, in applications where security is key, it is necessary to provide more trust in order to enhance decisions safety and quality.

For providing a better understanding and a basis for verification of the CNN, several approaches are known in state of the art.

A first approach is to use backpropagation-based mechanisms, which are directed on explaining the decisions of the CNN by producing so called saliency maps for the input vectors (e.g. images). A saliency map serves as an (intuitive) explanation for CNN classification decisions. In computer vision, a saliency map is defined as a 2D topological map that indicates visual attention priorities in a numerical scale. A higher visual attention priority indicates the object of interest is irregular or rare to its surroundings. The modeling of saliency is beneficial for several applications including image segmentation, object detection, image re-targeting, image/video compression etc. In particular, a layer-wise backpropagation (in the following abbreviated as LRP) may be used to generate such saliency maps. The paper "Bach, S., Binder, A., Montavon, G., Klauschen, F., Muller, K.R., Samek, W.: 'On pixel-wise explanations for non-linear classifier decisions by layer-wise relevance propagation'. PloS one 10 (2015) e0130140" proposes LRP to generate the explanations for classification decisions. However, experiments show that the LRP-generated saliency maps are instance-specific, but not class-discriminative. In other words, they are independent of class information. The explanations for different target classes, even randomly chosen classes, are almost identical. The generated maps recognize the same foreground objects instead of a class-discriminative one.

The work of Zhang et al (Zhang, J., Lin, Z., Brandt, J., Shen, X., Sclaroff, S.: Top-down neural attention by excitation backprop. In: European Conference on Computer Vision, Springer (2016) 543-559) discloses a formulation of the top down attention of a CNN classifier as a probabilistic winner-takes-it-all process. This paper, however, does not relate to a bottom-up learning. Further, this paper constructs a contrastive signal by negating the weights connecting the class. This application proposes other possibilities to construct the contrastive signal, e.g. represent the signal using all other classes. The normalization of saliency maps before subtraction depends on the maximum. The proposed application does not normalize the saliency maps because the conservative properties of LRP.

The work of Cao, C. et al. (Cao, C., Liu, X., Yang, Y., Yu, Y., Wang, J., Wang, Z., Huang, Y., Wang, L., Huang, C., Xu, W., et al.: Look and think twice: Capturing top-down visual attention with feedback convolutional neural networks. In: Proceedings of the IEEE International Conference on Computer Vision. (2015) 2956-2964) is able to produce class-discriminative attention maps. However, this work requires modifying the traditional CNNs by adding extra feedback layers and optimizing the layers during the backpropagation. So, there is a need for being able to provide saliency maps without any modifications on the CNN structure.

The classic saliency models are based on either blocks (rectangle patches) or regions (superpixels). Their hand-crafted features are often extracted using the intrinsic cues in images, e.g. the uniqueness, distinctiveness or rarity in a scene. However, in more challenging scenarios, their performance is not satisfying. Other approaches require labor intensive and time-consuming labeling processes. It is therefore a need in the art to provide an improved approach for generating saliency maps.

The research on saliency modeling is influenced by bottom up and top down visual features or cues. The bottom up visual attention (exogeneous) is triggered by stimulus, where a saliency is captured as the distinction of image locations, regions, or objects in terms of low level features or cues in the input image, such as color, intensity, orientation, shape, T-conjunctions, X-conjunctions, etc.

The visual bottom-up attention was modeled explicitly with specific neural network architecture or computational models.

For being able to analyze or inspect the relation between the individual (visual) input and their feature representations, especially the evolvement of the feature representations with increasingly deeper layers of the CNN, there is a need in the art to provide a better understanding of the CNN decisions.

The disadvantage of State of the Art backpropagation-based approaches is that they do not provide information about the inner neurons and layers and thus of the features of the CNN although they might be helpful to explain the final classification. For a better and more detailed understanding of the inner functioning of the CNN a class discriminative explanation of features would be helpful.

As mentioned earlier, the disadvantage of State of the Art methods for generating saliency maps is that they are not flexible enough. Especially so-called supervised methods require labor-intensive and time-consuming labeling process. Thus, it would be helpful that arbitrary images and especially images without labeling, may be used as input.

It is therefore an object of the present invention to provide a solution for improving the verifications processes of CNNs. Further, the technical analysis and monitoring of the neural network processes on a layer-wise level and with respect to the decision task classes should be improved as well. All objects, mentioned before, serve the general object that security of processes using or applying the CNNs should be improved.

This object is solved by a method for verifying a visual classification architecture of a Convolutional Neural Network (and classification decisions derived therefrom), by a verification unit, by a computer program and/or a computer program product according to the appended independent claims. Advantageous aspects, features and embodiments are described in the dependent claims and in the following description together with advantages.

In the following, the proposed technique is described with respect to the claimed verification method as well as with respect to the claimed verification unit. Features, advantages or alternative embodiments herein can be assigned to the other claimed objects (e.g. the computer program or a computer program product) and vice versa. In other words, claims for the verification unit can be improved with features described or claimed in the context of the methods and vice versa. In this case, the functional features of the method are embodied by structural units of the system and vice versa, respectively.

In one aspect the invention relates to a method for verifying a visual classification architecture of a Convolutional Neural Network (CNN) and its classification results. The method comprises:

- Accessing a memory with the CNN, being trained for a visual classification task into a set of target classes;
- Using the CNN for an input image and after a forward pass of the CNN, in a backward pass:

    o Applying a contrastive layer-wise relevance propagation algorithm or
    o Applying a Bottom Up Attention pattern, which is implicitly learned by the CNN, to verify a classification ability of the CNN;

    for providing a verification signal.

According to a preferred embodiment the verification signal is provided as a saliency map not only for each of the target classes but also for a feature in a specific CNN layer. The saliency map is instance-specific and class discriminative. This has the advantage, that the verification is more detailed and on a fine-grained level. It is noted that the saliency map with the saliency features detected by the neural network comprises a relation between the input image (regions or even pixels or properties) and the features learned in a particular layer of the CNN. In the forward pass of the classification of an image, the activation values of neurons of a layer are the features of the image in the layer. It is also called feature representation because the activation values (a vector) of a layer contains the information of content of images. For example, the salient features can vary between simple structures to semantic object parts, such as an organ, or a lesion or a cancerous structure in the input image, depending on the input image and the classification task.

According to another preferred embodiment, a set of pixel-wise saliency maps is generated for each individual neuron of each target class. This feature also improves detailedness of the verification result.

According to another preferred embodiment, the CLRP algorithm comprises, the steps of:

- Generating a first saliency map for each target class of the classification task by means of a backpropagation algorithm;
- Calculating a set of virtual classes for each target class, being opposite of the respective target class;
- Generating a second saliency map for the set of virtual classes by means of a backpropagation algorithm;
- Computing the differences between the first and the second saliency map for computing a final saliency map.

In further preferred embodiment, the calculation of the virtual class for a specific target class may be executed by:

- defining any other of the set of target classes (except the specific class) as virtual class or by
- defining all other target classes of the set of target classes (except the specific class) as virtual class or by
- constructing the virtual class by generating an additional class and connecting it with a last layer using weights, wherein the weights are the inverted weights of the forward pass.

In another preferred embodiment, applying the Bottom Up Attention pattern comprises:

- Collecting and storing all features of the CNN, a feature comprising all activations in a respective layer of the CNN for the input image;

- Creating a saliency map for each of the features.

With this, it is possible to verify the bottom up attention using the created list of saliency maps.

In another preferred embodiment, the visual classification task is a medical classification task in medical images in order to detect anomalies.

In another preferred embodiment, application of the CNN is only approved, if the provided verification signal is above a pre-configurable confidence threshold (representing error free decisions of the CNN).

According to another embodiment, when applying a Bottom Up Attention pattern for generating a saliency map for the features an amended and generalized type of backpropagation-based algorithms is used. Due to the fact that according to the invention a saliency map is not generated for the classes but for the features, the known backpropagation-based algorithms cannot be applied. Therefore, the backpropagation-based algorithms are amended. For example, the DeConvolutional algorithm, the gradient-based backpropagation algorithm and the guided backpropagation algorithm are amended to create a list of saliency maps for features (not for classes).

In this respect, the features are the activation values of neurons in a specific layer. A saliency map for a feature specifies which pixels of input images are important to the activation values.

In another preferred embodiment, the generated saliency maps are post processed and/or may be refined and/or an averaging and/or a thresholding may be applied.

In another aspect the present invention relates to a verification unit which is configured for verifying a visual classification architecture of a CNN, comprising:

- A memory with a CNN, being trained for a visual classification task into a set of target classes;
- A processor which is configured for using the CNN and wherein the processor is configured after a forward pass of the CNN, in a backward pass:

    o to apply a contrastive layer-wise relevance propagation algorithm or
    o to apply a Bottom Up Attention pattern, which is implicitly learned by the CNN, to verify a classification ability of the CNN,

- for generating a saliency map for each of the target classes.

The proposed method has the advantage that an additional check is possible whether it is secure to use the CNN for the particular automatic decision (classification task). The working of the trained CNN is no longer a black box, but its reasoning may be made transparent and "retraceable". Further, the input images need not to be specific or need not be prepared in a certain manner (e.g. by labeling). Thus, the method is much more flexible than known ones.

The bottom-up mechanism proposes a set of salient image regions or pixels, with each region represented by a pooled convolutional feature vector. Generally, deep features are the response images of convolution, batch normalization, activation, and pooling operations in a series of layers in a convolutional neural network. Such response images provide semantic information about the image. Initial layers present low level features or cues such as edges, and a higher level abstract is obtained as a function of layer number. Latter layers provide higher level of semantic information such as a class of objects.

In the following the terms used within this application are defined.

The verification signal is to be construed as electronic signal or dataset, representing a root cause in the image for the respective decision. The verification signal may be provided in different formats, e.g. as overlay in the input image and thus in a graphical format (e.g. bounding box or highlighted image areas or fields). Also, the verification signal may be post processed and provided as binary signal, representing a verification status, simply signaling a "verified decision" or a "non-verified decision". The verification signal may be provided on an output entity, which may be portion or window on a monitor. The verification signal may be provided on the same monitor as the input signal. The verification signal is configured to provide a technical basis for verification of the CNN architecture and its logic and decisions, respectively.

The contrastive layer-wise relevance propagation is strategy which will be explained in more detail below and in the detailed description. The contrastive layer-wise relevance propagation may be implemented as application or computer program.

Generally, the invention relates to Deep Learning as a part of machine learning that uses multiple layers of computer processing entities, called neurons, wherein the neurons are interconnected and exchange messages between each other. The connections have numeric weights that can be tuned based on experience, making neural networks adaptive to inputs and capable of learning.

One of the most popular types of deep learning architecture is a Convolutional Neural Network (CNN) is disclosed in Simonyan, Karen ; Zisserman, Andrew: Very Deep Convolutional Networks for Large-Scale Image Recognition. In:

CoRR, abs/1409.1556 (2014), and Szegedy, Christian et al. "Going deeper with convolutions." 2015 IEEE Conference on Computer Vision and Pattern Recognition (CVPR) (2015): 1-9; as well as in He, K., Zhang, X., Ren, S., & Sun, J. (2016). Deep residual learning for image recognition. In Proceedings of the IEEE conference on computer vision and pattern recognition (pp. 770-778). For more detailed technical information, it is referred to these documents, their content is incorporated by reference.

A CNN is a multi-layered image-processing unit comprising convolutional, pooling and rectified linear unit (ReLU) layers. These layers can be arranged in any order as long as they satisfy the input/output size criteria.

A Convolutional Neural Network (CNN) can be thought of as a layered image-processing pipeline designed to perform a particular task, e.g. a classification task of a medical image. The goal of the pipeline is to take an image as input, perform mathematical operations and provide a high-level user-friendly response. The processing within the network is sequential in nature: i.e., each layer in the network takes input from the layer(s) above it, does some computation before passing the resulting output to the next layer(s).

Each layer is composed of "neurons" that are connected to "neurons" of other (in most cases adjacent) layers. Each connection has a numeric weight associated with it that signifies its importance.

There are two main steps when working with CNNs: training and testing. Before a CNN can be used for a task, it needs to be trained for that task. In the training phase, the CNN is provided with a list of objects that need to be detected and classified by the network. It is also given a collection of images where each image is associated with a set of user-defined concepts (ground-truth labels based on and not exceeding the object category list). The goal is to tune the connection weights in the network in such a manner so as to produce an output that matches the ground-truth labels as best as possible. This is achieved by combining the weights, network output and ground-truth labels to design a cost function where the cost is zero when network object categorization output matches the image ground-truth labels. Thus, the weights are tuned to bring the cost down as much as possible, which in turn leads to improved accuracy (which is a measurement of how closely the network output and ground-truth labels match). Once the weights have been tuned to get the best possible results for the training data, one can simply use it for testing by passing an image and getting an output.

A CNN includes an ordered stack of different types of layers e.g. convolutional, pooling, ReLU (rectified linear unit), fully connected, dropout, loss, etc. Each layer takes input from one or more layers above it, processes the information and passes the output to one or more layers below it. Generally, a layer takes input from the layer immediately above it and passes the output to the layers immediately below. But it can certainly be designed to take input and pass output from multiple layers.

Each layer comprises of a set number of image filters. The output of filters from each layer is stacked together (in the third dimension). This filter response stack then serves as the input to the next layer(s).

For classification, the result of the fully connected layers is processed using a loss layer that generates a probability of how likely the object belongs to a specific class.

The memory may refer to drives and their associated storage media providing nonvolatile storage of machine readable instructions, data structures, program modules and other data for the computer. The memory may include a hard disk, a removable magnetic disk and a removable (magneto) optical disk. Those skilled in the art will appreciate that other types of storage media, such as magnetic cassettes, flash memory cards, digital video disks, Bernoulli cartridges, random access memories (RAMs), read only memories (ROM), and the like, may be used instead of, or in addition to, the storage devices introduced above.

The training of the CNN is not restricted to a specific type of training (supervised, unsupervised). The training data may be stored locally or externally on another memory.

The steps of applying/using the CNN and applying the algorithms are executed on a computer. In particular, a processor (relating to a processing circuitry or hardware) is provided for execution of the above mentioned steps and functions. However, it is also possible that these steps are executed on dedicated hardware (e.g. a graphical processing unit GPU) and may be executed in a distributed manner on different computing entities (in data connection) in order to save computing resources.

The Bottom Up Attention pattern is a mechanism which is implicitly learned by the CNN. Traditional bottom-up strategies aim to regularize the network training and have been modeled.

Generally, there is a network connection (e.g. a local network LAN or WLAN or internet protocol based connection or wired connection) between different computing entities, used for the method, in particular, an input entity, an output entity, the memory and/or the processor, the verification unit.

In another aspect the invention relates to a computer program product comprising a computer program, the computer program being loadable into a memory unit of a computer, including program code sections to make the computer execute the method for verification CNN decisions according to an aspect of the invention, when the computer program is executed in said computer.

In another aspect the invention relates to a computer-readable medium, on which program code sections of a computer program are stored or saved, said program code sections being loadable into and/or executable in a computer to make the computer execute the method for verification CNN decisions according to an aspect of the invention, when the program code sections are executed in the computer.

The realization of the invention by a computer program product and/or a computer-readable medium has the advantage that already existing computers in the application field, servers or clients can be easily adopted by software updates in order to work as proposed by the invention.

The properties, features and advantages of this invention described above, as well as the manner they are achieved, become clearer and more understandable in the light of the following description and embodiments, which will be described in more detail in the context of the drawings. This following description does not limit the invention on the contained embodiments.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

Fig. 1     is a schematic illustration of a convolutional neural network, constructed and operative in accordance with a preferred embodiment of the disclosed technique;

FIG. 2     is another more detailed schematic illustration of a fully connected deep convolutional neural network, which has been trained to classify the input image in two different target classes and operative in accordance with another embodiment of the disclosed technique;

FIG. 3     is a schematic illustration of a system for using a deep convolutional neural network for providing an output and operative in accordance with a further embodiment of the disclosed technique;

Fig. 4     is a schematic block diagram with electronic units for executing a verification method according to a preferred embodiment of the present technique;

Fig. 5     shows a calculated verification signal in more detail for different layers of the deep convolutional neural network;

Fig. 6     shows an overview of the CLRP algorithm for two exemplary target classes, representing ZEBRA and ELEPHANT;

Fig. 7     shows four different input images of multiple objects, which are classified using a neural network implementation and respective saliency maps which are provided for the two relevant classes, generated by LRP and by CLRP algorithm and

Fig. 8     is a simplified flow chart of a method according to a preferred embodiment of the proposed technique.

DETAILED DESCRIPTION OF THE DRAWINGS

The disclosed technique overcomes the disadvantages of the prior art by providing a method and a system for verifying the architecture and inner working of a deep neural network for an image classification task.

The proposed technique is implemented and provided as a computer program. A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

In the following a general explanation of the functioning and architecture of a convolutional neural network is given before going into the details of the present invention. In general, with the proposed technique, the architecture and the training of the Convolutional Neural Network CNN may be verified by means of providing a verification signal vs.

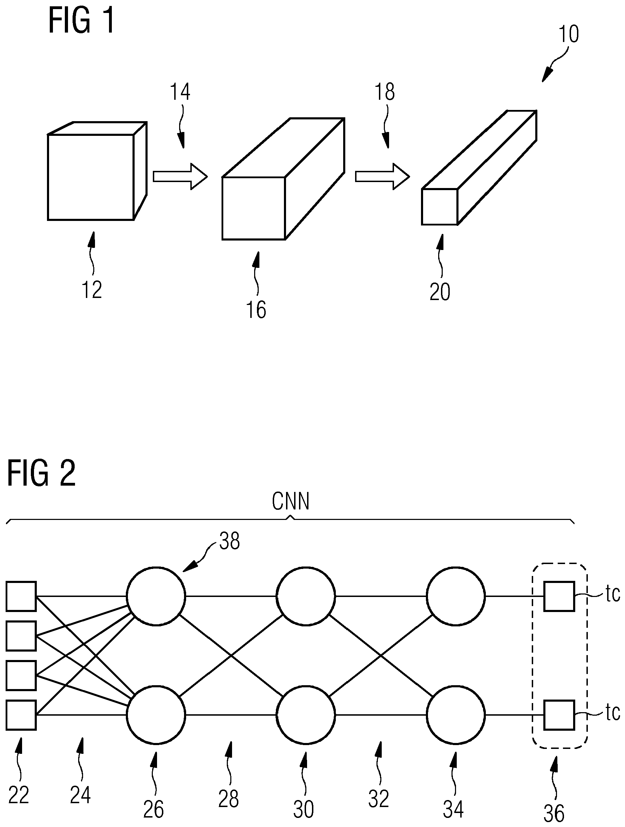

Reference is now made to FIGS. 1, which are schematic illustrations of a typical known Convolutional Neural Network CNN, generally referenced as 10. The operation and construction of the CNN 10 is verified in accordance with an embodiment of the disclosed technique. FIG. 1 depicts an overview of CNN 10. With reference to FIG. 1, CNN 10 includes an input image 12 to be classified, followed by e.g. first and/or second convolutional layers 14 and 18 with respective outputs 16 and 20. It is noted that CNN 10 can include more, or less, convolutional layers. The output of second convolutional layer 20 may e.g. then be vectorized in vectorizing layer. A vectorization output may be fed into further layers of a fully connected, neural network.

In the example set forth in FIG. 2, a vectorized input 22 is used. In the fully connected neural network of CNN 10 there may for example be three fully connected layers 26, 30 and 34 (more, or less, layers are possible) and the output vector 36 with (in this simplified example) two classification classes tc. Reference numeral 38 represents a neuron in one specific layer. Each of fully connected layers 26, 30 and 34 comprises a variable number of linear, or affine, operators - which are referenced in Fig. 2 with 24, 28 and 32 - potentially followed by an e.g. nonlinear or sigmoid activation function. The last fully connected layer 34 is typically a normalization layer so that the final elements of an output vector 36, which refer to the target classification classes tc are bounded in some fixed, interpretable range. The parameters of each convolutional layer and each fully connected layer are set during a training (i.e., learning) period of CNN 10.

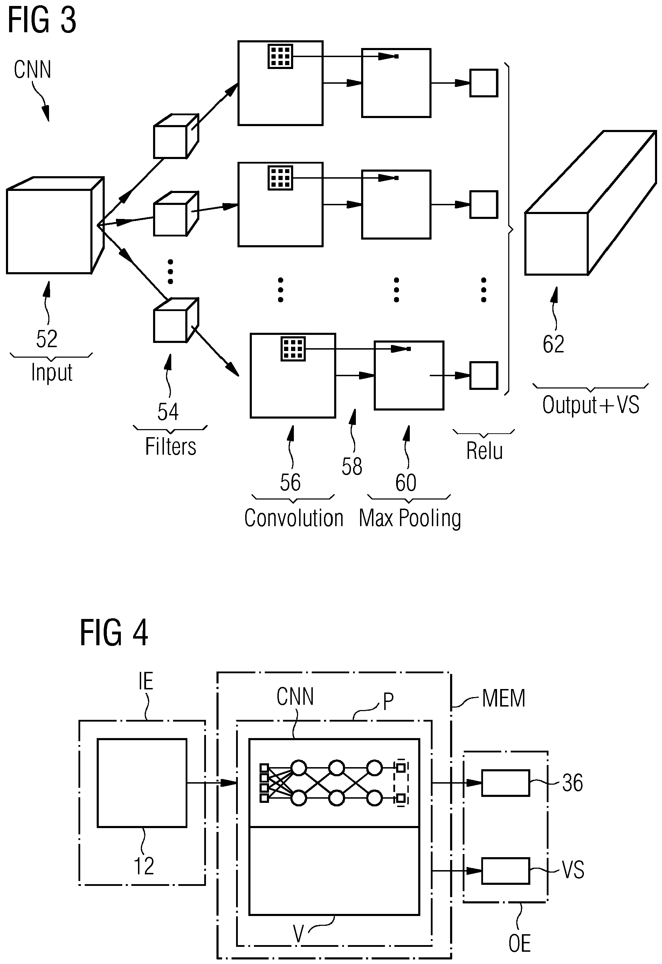

The structure and operation of each of the convolutional layers and the fully connected layers is further detailed with reference to FIG. 3. Each input to a convolutional layer is an input image, which is referenced in FIG. 3 with 52. For example, the input image may be a medical image (2D or 3D) which is to be classified with respect to healthy and disease structures. The input 52 may be convolved with filters 54 that are set in the training stage of CNN 10. Each of the filters 54 may e.g. be convolved with the layer input 52 to generate a two-dimensional (2D) matrix 56. Dependent on the respective classification task, subsequently or in other layers, an optional max pooling operation 58 and/or an optional ReLU operation (by means of a Rectified linear unit) may be applied. The output of the neural network CNN 10 is an output vector 62 with probabilities for the different target classes (in the given example above: two; e.g. a prediction 0,3% for the normal class and 0,7% for the abnormal class). The proposed solution can provide supportive evidence for these predictions by also providing the calculated verification signal vs.

According to the invention the output 62 does not only comprise the output vector with the classification target classes tc, but also a verification signal vs. In particular, the verification signal vs represents the route cause and thus the reason why the input image has been classified with a 0,7% probability to be abnormal. In particular, the respective image portions and parts may be highlighted or marked, which are causal for the CNN decision result and are causal for a processing of a specific neuron of a specific (inner) layer of the CNN as well. Thus, not only the output layer is considered, but also all inner layers on a detailed level.

Each of convolutional layer outputs, and fully connected layer outputs, details the image structures (i.e., features) that best matched the filters of the respective layer, thereby identifying those image structures. In general, each of layers in a convolutional neural network CNN detect image structures in an escalating manner such that the deeper layers detect features of greater complexity. For example, it has been empirically demonstrated that the first convolutional layer detects edges, and the second convolutional layer, which is deeper than first layer, may detect object attributes, such as curvature and texture. It is noted that CNN 10 (FIG. 1) can include other numbers of convolutional layers, such as a single layer, four layers, five layers and the like.

If such a CNN has been trained and is to be used on a particular input image to be classified, it may turn out, that the decisions are not 100% adequate and that the CNN may provide mistakes. Therefore, the proposed technique provides a measure for verification of a CNN. The verification improves security and quality of the process in which the CNN is involved or applied (e.g. a medical diagnostic process). In the following the proposed verification technique is explained with respect to a deep convolutional neural network (DCNN). However, the disclosed technique is also applicable to other types of artificial neural networks (besides DCNNs). In particular, in shallow networks, it is possible to get discriminative information directly using LRP. The CLRP proposed in this application still works. In deep neural networks (not necessary CNN), LRP does not work, and CLRP works very well.

FIG. 4 shows schematic drawing of a verification system. The system comprises an input entity IE for providing an image to be analyzed (classification task) and an output entity OE for providing the classification result 36 and the verification signal vs. In a preferred embodiment, the input entity IE and the output entity OE may be integrated in one common unit, e.g. a graphical device, like a monitor. Other media may be used, too. The entities IE, OE are connected electronically (data link, like network connection) to a memory MEM in which the processing circuitry P may be implemented. The memory MEM or a particular portion thereof may be responsible for storing the trained deep CNN. Further, a verification unit V is provided for executing the verification as mentioned herein in order to provide a verification signal vs in order to verify and check the CNN decisions for every neuron in the different layers with respect to the target class tc. For a person skilled in the art, of course, the architecture may be amended without leaving the scope of this invention. For example, the processor P, the verification unit V and the memory MEM may also be separate units and deployed on different hardware, being in data exchange.

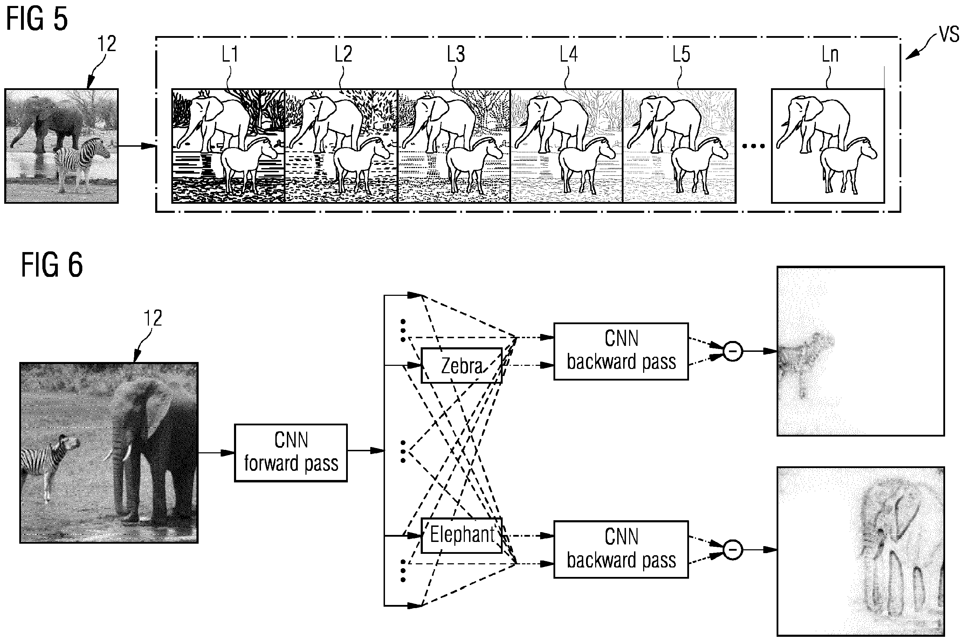

FIG. 5 shows another schematic representation of the calculated verification signal vs in more detail. The input image 12 shows semantic content which is to be classified according to a specific classification task at hand. In the simplified example, an elephant and a zebra is represented in the foreground and the classification task is to identify the animals in the image and to separate them from each other and from other (background) structures. So, for both of the target classes tc (here: elephant and zebra) the verification signals vs is calculated for each of the layers L1,...Ln.

As can be seen in FIG. 5, the relevance of the pixels in the input image 12 are shown to the feature representation in each layer L1-Ln. In experiments, four different generalized methods are used, to compute the relevance value, namely, Deconv: DeConvNets Visualization, vaGrad: vanilla Gradient Visualization, GuidBP: Guided Backpropagation, LRP: Layer-wise Relevance Propagation. The shallow layers are in left and deep ones in right. The experiments showed that for each of the four methods, a trained VGG16 model shows a bottom-up attention mechanism. For comparison, if the methods are applied to an untrained VGG16 model, the visualization does not show such a bottom-up attention mechanism.

In the following the known layer-wise relevance propagation (LRP in short) is explained in more detail in order to show the amendments which have been applied according to the technique presented herein.

Each neuron in DCNNs represents a non linear function

$$X_{i+1} = \phi(X_i W_i + b_{i+1}),$$

where $\Phi$ is an activation function and $b_{i+1}$ is a bias vector for the neurons $X_{i+1}$. The inputs of the nonlinear function corresponding to a neuron are the activation values of the previous layer $X_i$ or the raw input of the network. The output of the function are the activation values of the neuron $X_{i+1}$. The whole network is composed of the nested nonlinear functions. To identify the relevance of each input variables, the LRP approach (for details see paper Bach et al., mentioned above in the prior art section) propagates the activation value from a single class-specific neuron back into the input space, layer by layer. The activation value is taken before softmax normalization. In each layer of the backward pass, given the relevance score $R_j$ of the neurons $X_{i+1}$, the relevance $R_i$ of the neurons $X_i$ are computed by redistributing the relevance score using local redistribution rules. The most often used rules are the $z^+$ -rule and the $z^\beta$ -rule, which are defined as follows:

$$z^+ - \text{rule:} R_i = \sum_j \frac{x_i w_{ij}^+}{\sum_{i'} x_{i'} w_{i'j}^+} R_j$$

$$z^\beta - \text{rule:} R_i = \sum_j \frac{x_i w_{ij} - l_i w_{ij}^+ - h_i w_{ij}^-}{\sum_{i'} x_{i'} w_{i'j} - l_i w_{i'j}^+ - h_i w_{i'j}^-} R_j$$

and the interval [l, h] is the input domain.

In our work, we provide a theoretical foundation for the fact that in deep convolutional rectifier neuron network, the ReLU masks and Pooling Switches decide the pattern visualized in the explanation, which is independent of class information. That is the reason why the explanations (saliency maps) generated by LRP on DCNNs are not class-discriminative. The analysis also explains the non-discriminative explanations generated by other backpropagation approaches, such as the DeConvNets Visualization, The vanilla Gradient Visualization and the Guided Backpropagation.

Therefore, we amended and generalized the above mentioned known backpropagation-based algorithms to provide a new algorithm, called Contrastive Layer-wise Relevance Propagation, in short CLRP, for getting a class discriminative explanation in the form of a saliency map.

Contrastive Layer-wise Relevance Propagation CLRP

Before introducing our CLRP, we first discuss the conservative property in the LRP. In a DNN, given the input X = {$x_1$, $x_2$, $x_3$, ···, $x_n$}, the output Y = {$y_1$ , $y_2$ , $y_3$ , ···, $y_m$}, the score $S_{yj}$ (activation value) of the neuron $y_j$ before softmax layer, the LRP generate an explanation for the class y j by redistributing the score $S_{yj}$ layer-wise back to the input space. The assigned relevance values of the input neurons are R = {$r_1$ , $r_2$ , $r_3$ , ···, $r_n$}. The conservative property is defined as follows:

Definition 1. The generated saliency map is conservative if the sum of assigned relevance values of the input neurons is equal to the score of the class-specific neuron,

$$\sum_{i=1}^n r_i = S_{yj}$$

In this section, we consider redistributing the same score from different class-specific neurons respectively. The assigned relevance R are different due to different weight connections. However, the non-zero patterns of those relevance vectors are almost identical, which is why LRP generate almost the same explanations for different classes. The sum of each relevance vector is equal to the redistributed score according to the conservative property. The input variables that are discriminative to each target class are a subset of input neurons, i.e., X dis ⊂ X. The challenge of producing the explanation is to identify the discriminative pixels X dis for the corresponding class. In the explanations of image classification, the pixels on salient edges always receive higher relevance value than other pixels including all or part of Xdis. Those pixels with high relevance values are not necessary discriminative to the corresponding target class. We observe that X dis receive higher relevance values than that of the same pixels in explanations for other classes. In other words, we can identify X dis by comparing two explanations of two classes. One of the classes is the target class to be explained. The other class is selected as an auxiliary to identify X dis of the target class. To identify X dis more accurately, we construct a virtual class instead of selecting another class from the output layer.

We propose at least two ways to construct the virtual class. The overview of the CLRP are shown in FIG. 6. For each predicted class, the approach generates a class-discriminative explanation by comparing two signals. The dash-dotted line (in Fig. 6 in the upper backward pass: the lower two lines and in the lower backward pass: the upper lines) means the signal that the predicted class represents. The dotted line (in Fig. 6 in the upper backward pass: the upper two lines and in the lower backward pass: the lower lines) models a dual concept opposite to the predicted class. The final explanation is the difference between the two saliency maps that the two signal generate.

We describe the CLRP formally as follows. The jth class-specific neuron y j is connected to input variables by the weights W = {W1 , W2 , ⋯, Wi-1 , Wij } of layers between them, where W i means the weights connecting the (i - 1)th layer and the ith layer, and Wij means the weights connecting the (i - 1)th layer and the jth neuron in the ith layer. The neuron y j models a visual concept O. For an input example X, the LRP maps the score S y j of the neuron back into the input space to get relevance vector R = f LRP (X, W, Syj).

We construct a dual virtual concept $\overline{O}$ which models the opposite visual concept to the concept O. For instance, the concept O models the zebra, and the constructed dual concept $\overline{O}$ models the non-zebra. One way to model the virtual concept $\overline{O}$ is to select all classes except for the target class representing O. The concept $\overline{O}$ is represented by the selected classes with weights W = {W1, W2 , ⋯, Wi-1 , Wi{-j}}, where Wi{-j} means the weights connected to the output layer excluding the jth neuron. E.g. the dot-dashed lines in FIG. 6 are connected to all classes except for the target class zebra. Next, the score Syj of target class is uniformly redistributed to other classes. Given the same input example X, the LRP generates an explanation R dual = f LRP (X, W, Syj) for the dual concept.

The Contrastive Layer-wise Relevance Propagation is defined as follows:

$$R_{CLRP} = \max(0, (R - R_{dual})),  \text{(Equation 2)}$$

where the function max(0, X) means replacing the negative elements of X with zeros. The difference between the two saliency maps cancels the common parts. Without the dominant common parts, the non-zero elements in R CLRP are the most relevant pixels Xdis. If the neuron y j lives in an intermediate layer of a neural network, the constructed R CLRP can be used to understand the role of the neuron.

The other way to model the virtual concept $\overline{O}$ is to negate the weights W ij . The concept $\overline{O}$ can be represented by the weights W = {W1 , W2 , ⋯, Wi-1 , -1*Wij}. All the weights are same as in the concept O except that the weights of the last layer Wij are negated. In the experiments section, we call the first modeling method CLRP1 and the second one CLRP2. The contrastive formulation in the paper "Zhang, J., Lin, Z., Brandt, J., Shen, X., Sclaroff, S.: 'Top-down neural attention by excitation backprop'. In: European Conference on Computer Vision, Springer (2016) 543-559" can be applied to other backpropagation approaches by normalizing and subtracting two generated saliency maps. However, the normalization strongly depends on the maximal value that could be caused by a noisy pixel. Based on the conservative property of LRP, the normalization is avoided in the proposed CLRP.

We conduct experiments to evaluate our proposed approach. The first experiment aims to generate class-discriminative explanations for individual classification decisions.

In the experiments, the LRP, the CLRP1 and the CLRP2 are applied to generate explanations for different classes. The experiments are conducted on a pre-trained VGG16 Network (for more details see Simonyan, K., Zisserman, A.: 'Very deep convolutional networks for large-scale image recognition'; arXiv preprint arXiv:1409.1556 (2014). The propagation rules used in each layer are the same as mentioned above, explained with respect to LRP. We classify the images of multiple objects. The explanations are generated for the two most relevant predicted classes, respectively.

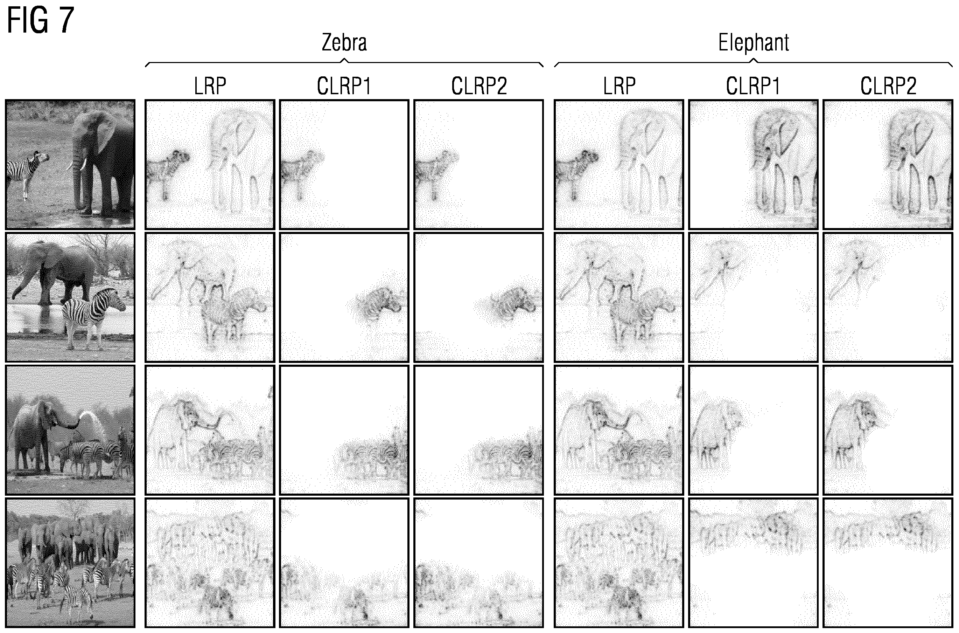

FIG. 7 shows the explanations for the two classes (i.e., Zebra and African_elephant). Generally, in FIG. 7 the images of multiple objects are classified using VGG16 network pre-trained on ImageNet. The explanations for the two relevant classes are generated by LRP and CLRP. The CLRP generates class-discriminative explanations, while LRP generates almost same explanations for different classes (here: Zebra, elephant). Each generated explanation visualizes both Zebra and African _elephant, which is not class-discriminative. By contrast, both CLRP1 and CLRP2 only identify the discriminative pixels related to the corresponding class. For the target class Zebra, only the pixels on the zebra object are visualized. Even for the complicated images where a zebra herd and an elephant herd co-exist, the CLRP methods are still able to find the class-discriminative pixels.

With respect to Fig. 6 and 7 it is to be noted, that the originally automatically calculated figures include a large black portion, therefore the images have been converted into a schematic representation. Thus, the text is more adequate and relevant compared to the figures.

We evaluate the approach with a large number of images with multiple objects. The explanations generated by CLRP are always class-discriminative, but not necessarily semantically meaningful for every class. One of the reasons is that the VGG16 Network is not trained for multi-label classification. Other reasons could be the incomplete learning and bias in the training dataset.

The implementation of the LRP is not trivial. The one provided by their authors only supports CPU computation. For the VGG16 network, it takes the 30s to generate one explanation on an Intel Xeon 2.90GHz×6 machine. The computational expense makes the evaluation of LRP impossible on a large dataset. We implement a GPU version of the LRP approach, which reduces the 30s to 0.1824s to generate one explanation on a single NVIDIA Tesla K80 GPU. The implementation alleviates the inefficiency problem and makes the quantitative evaluation of LRP on a larger dataset possible.

In the experiment, we proofed that it is possible to study the difference among neurons in a single classification decision. The neurons of low layers may have different local receptive fields. It was further proofed that different neurons focus on different parts of images.

The difference between them could be caused by the different input stimuli. We visualize high-level concepts learned by the neurons that have the same receptive fields, e.g., a single neuron in a fully connected layer. For a single test image, the LRP and the CLRP2 are applied to visualize the stimuli that activate a specific neuron. We do not use CLRP1 because the opposite visual concept cannot be modeled by the remaining neurons in the same layer. In the VGG16 network, we visualize the 8 activated neurons x 1-8 from the fc1 layer.

It was further proofed that different neurons focus on different parts of images. This information will be provided in the verification signal vs, to make the CNN decision transparent for the user and retraceable in the input image.

Another aspect of the proposed technique relates to using the concept of bottom up attention for feature evaluation. Generally, the calculation of a verification signal vs as a result dataset makes it possible to investigate how the relationship between individual input images and their feature representations evolves with increasingly deeper layers. With the technique proposed herein, it is possible to analyze and verify not only the output layer of the CNN, but also the inner layers in order to get a much more detailed understanding (represented in the verification signal vs).

Thus, given the verification signal vs, the CNN decisions may be subject to a verification, check or additional analysis. Given a single input image, for the representation of each layer, we find the stimuli from the input image relevant to the representation. By comparing the responsible stimuli pattern corresponding different layers, we can understand the difference in the feature representations in different layers.

As mentioned in the background section, the classic saliency models are not satisfying with respect to performance and flexibility; the latter, because a time-consuming labeling process has to be executed beforehand. The present technique overcomes these problems.

Known methods for explaining classification decisions. Like the vanilla Gradient Visualization, the Guided Backpropagation and the LRP identify the gradient-based values as the relevance of each pixel to a given class. They map the class-specific score to the input space $f(CNN, S_C(I_O))$, given an image $I_O$, a class c and the output score corresponding to class c is $S_C(I_O)$, which is produced with rectifier convolutional neural network. The predicted score is more easily affected by the pixels with high gradient values. To understand how the input stimuli affect the feature representation, we generalize the methods by replacing the class-specific score with the feature activations in intermediate layers. For the feature activations of each layer, the derivatives of them with respect to input pixels are computed as relevance values.

Given the feature representation Xn of an intermediate layer, we compute the gradients of pixels Ri for each activation $x_i \in Xn$. The gradients are weighted by the corresponding activation xi and aggregated for each pixel respectively. The final relevance value is defined as

$$R = \sum_i^{|x_n|} R_i = \sum_i^{|x_n|} x_i * f(CNN_{1-i,x_i}) \qquad \text{(Equation 1)}$$

where the mapping f means the methods introduced before, namely, the DeConvNets, the vanilla Gradient or the Guided Backpropagation. They map the activation value back to input space; $CNN_{1-i}$ means the parameters and the structure information of first i layers in the CNN. By visualizing the normalized relevance values, we can explore the difference between feature representations of all layers.

The LRP method propagates the score Sc(Io) back into input space layer-by-layer. In each layer, LRP redistributes score according to the specific propagation rules, such as the $z^+$-rule and the $z^\beta$-rule. The relevance value assigned to each pixel by LRP means their relevance to the predicted class. LRP quantifies the contribution of pixels to a class-specific output score. Similarly, we can apply the LRP method to quantify the contribution of each pixel to the learned feature representation. We could apply LRP to get the importance value of pixels to feature representations.

Generally, the high values of the intermediate layers can date back to the pixels on the boundary of the objects of the input image. The feature maps in more deeper layers do not show visually recognizable patterns, because the sizes thereof are small. The values in deep layers code the information from intermediate layers. These and the feature representations of intermediate layers are influenced by almost the same pixels on the input images, namely, the pixels on the boundary of the foreground objects. As the convolutional operations in VGG16 going deeper layer-by-layer, the computed feature representation focus more and more on the boundary of the foreground objects. The well-trained deep convolutional neural networks show the bottom-up attention.

From experiments we learned that that filters learned in CNNs contain large amounts of edge-detection filters and blurring filters, which hardly exist in an untrained model. The edges mentioned in this section mean not only the salient edges in input images but also the salient positions where the activation value are, in contrast to that of the surrounding neighborhood. The convolutional operations with blurring filters blur the images or feature maps so that the local low contrastive information (local edges) are lost. However, most salient edges (the contour of the salient objects) are kept. The convolutional results with edge-detection filters focus on the salient edges. After several convolutional layers, the kept activations live on the boundary of the most salient objects. For an untrained model, only very limited number of such filters exist. Besides, the found similar filters deviate more from the meaningful filters.

In the following it will be described how to model visual saliency based on the bottom-up Attention in CNNs. While the features focus on local saliency in low layers, they extract global saliency (high-level salient foreground object) in deep layers. Using the Guided Backpropagation approach, we compute the saliency maps that correspond to features in a deep layer using the method described in equation 1, above. The computed saliency map focuses more on the boundary of the salient objects. We simply process the saliency maps with Gaussian Blur. The processed saliency maps are taken as final saliency maps. We use the off-the-shelf deep CNNs pre-trained on the ImageNet. The fully connected layers require a fixed size of the input image. By removing the layers, the remaining fully convolutional layers can produce full resolution saliency maps.

The proposed method does not require any fully or weakly supervised information. In particular, the verification method, presented here does not require category labels, bounding box labels and pixel-wise segment labels.

To further refine the saliency map, in a preferred embodiment, the image is segmented, using superpixels. For each superpixel, it is possible to average the saliency value on all pixels, and then to apply a thresholding on the saliency map to remove the low saliency, which removes the noisy values on saliency maps. Another option is to average the saliency maps of one image and its noisy variants. The post-process does improve the performance on the saliency detection task.

With using the Bottom Up Attention mechanism, as described above, it is possible to detect salient objects. In experiments, we compared their performance on the saliency detection task, and show the difference between different layers and different convolutional networks, regarding their bottom-up attention ability. By detecting salient objects, we verify the effectiveness of the bottom-up attention of the pre-trained CNNs. The competitive detection performance indicates that the bottom-up attention is an intrinsic ability of CNNs.

Concerning the implementation details, pre-trained model were taken from torchvision module in Pytorch, namely, AlexNet, VGGNet, ResNet. The fully connected layers of these CNNs are removed. The raw images without resizing are taken as the input for the forward passes. The feature representations of the last layer before the fully connected layers are computed. For each feature representation (activations in each layer), we create a saliency map whose values indicates how relevant is each pixel to the feature representation. The saliency maps are then processed with Gaussian Blur. The pixels relevant to a high-layer feature often lie on salient foreground objects. Namely, the values of the saliency maps correspond to the saliency of each pixel of the corresponding input image.

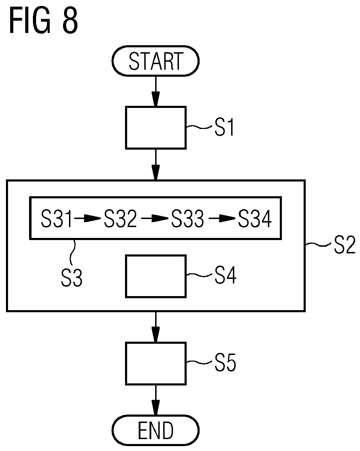

The general process for providing a verification dataset will be explained below with respect to FIG. 8.

FIG. 8 shows a flow chart according to a preferred embodiment of the present invention. After Start of the verification method, in step S1 the memory MEM is accessed for using the stored trained CNN for an image classification task. In another step an input image is received and in step S2 the CNN is applied on the input image. During the execution phase two alternative sub steps S3 or S4 may be used, namely:

- Applying a contrastive layer-wise relevance propagation algorithm CLRP in step S3 or
- Applying a Bottom Up Attention pattern BUAP, which is implicitly learned by the CNN in the execution phase of the CNN (not in the training phase) in step S4

for providing a verification signal vs in step S5. After this, the method may be reiterated or may end.

In sum, the verification calculated by the verification signal vs provides a better understanding of individual decisions of a CNN by means of applying the contrastive backpropagation algorithm, as explained above. By using the contrastive backpropagation, the verification method becomes less computational expensive (in particular no optimization steps are necessary) and offers a better understanding of the trained CNN. Moreover, it can help to debug the CNN by adapting the architecture and/or the training procedure of the CNN.

According to the method and units described above, it is possible to identify the relevance of each input by redistributing the prediction score back into the input space, layer by layer.

A visual classification task can also be an industry classification task in order to detect anomalies of images generated by a camera, video or other visual image generating devices of products, like a layer of an object produced by additive manufacturing or visualization charts of sensor data.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantageous which are described with respect to a particular embodiment of present invention or with respect to a particular figure are, wherever applicable, also advantages of other embodiments of the present invention. Any reference signs in the claims should not be construed as limiting the scope.

**Claims**

1. Computer-implemented method for verifying a visual classification architecture of a convolutional neural network (CNN), comprising the method steps of:

   - Accessing (S1) a memory (MEM) with a convolutional neural network (CNN), being trained for a visual classification task into a set of target classes (tc);
   - Using (S2) the convolutional neural network (CNN) for an input image (12) and after a forward pass of the convolutional neural network (CNN), in a backward pass;
   - Applying (S3) a contrastive layer-wise relevance propagation algorithm (CLRP) or
   - Applying (S4) an implicitly learned Bottom Up Attention pattern (BUAP), to verify a classification ability of the convolutional neural network (CNN) for providing (S5) a verification signal (vs).

2. Method according to claim 1, wherein the verification signal (vs) is provided as a saliency map for each feature on each layer of the convolutional neural network (CNN).

3. Method according to any of the preceding claims, wherein by applying (S3) the contrastive layer-wise relevance propagation algorithm (CLRP) class discriminative and instance-specific saliency maps are generated.

4. Method according to any of the preceding claims, wherein for applying (S4) an implicitly learned Bottom Up Attention pattern (BUAP), a deconvolutional CNN algorithm, a gradient backpropagation algorithm or a layer-wise backpropagation algorithm are amended in order to generate saliency maps for features and not for classes.

5. Method according to any of the preceding claims, wherein the CLRP algorithm (S3) comprises, the steps of:

   - Generating (S31) a first saliency map for each target class (tc) of the classification task by means of a backpropagation algorithm;
   - Calculating (S32) a set of virtual classes for each target class (tc), being opposite of the respective target class (tc);
   - Generating (S33) a second saliency map for the set of virtual classes by means of a backpropagation algorithm;
   - Computing (S34) the differences between the first and the second saliency map for computing a final saliency map.

6. Method according to the directly preceding claim, wherein calculating the virtual class for a specific target class (tc) is executed by:

   - defining any other of the set of target classes as virtual class or by
   - defining all other target classes of the set of target classes as virtual class or by
   - constructing the virtual class by generating an additional class and connecting it with a last layer using weights, wherein the weights are the inverted weights of the forward pass.

7. Method according to any of the preceding claims, wherein applying (S4) the Bottom Up Attention pattern (BUAP) comprises:

   - Collecting and storing all features of the CNN, wherein a feature comprises all activations in a respective layer of the CNN for the input image;
   - Creating a saliency map for each of the features.

8. Method according to any of the preceding claims, wherein the visual classification task is a medical classification task in medical images in order to detect anomalies.

9. Method according to any of the preceding claims, wherein application of the convolutional neural network (CNN) is only approved, if the provided verification signal (vs) is above a pre-configurable confidence threshold.

10. Method according to any of the preceding claims, wherein when applying a Bottom Up Attention pattern for generating a saliency map a guided backpropagation algorithm is used.

11. Method according to any of the preceding claims, wherein the generated saliency maps are post processed and/or may be refined and/or an averaging and/or a thresholding may be applied.

12. A verification unit (V) which is configured for verifying a visual classification architecture of a convolutional neural network (CNN), comprising:

   - A memory (MEM) with a convolutional neural network (CNN), being trained for a visual classification task into a set of target classes (tc);
   - A processor (P) which is configured for using the convolutional neural network (CNN) and wherein the processor (P) is configured after a forward pass of the CNN, in a backward pass:
   - to apply a contrastive layer-wise relevance propagation algorithm (CLRP) or
   - to apply a Bottom Up Attention pattern (BUAP), which is implicitly learned by the CNN
   - for generating a saliency map for each of the target classes (tc).

13. A computer program product comprising program elements which induce a computer to carry out the steps of the method for verifying a visual classification architecture of a convolutional neural network (CNN) according to one of the preceding method claims, when the program elements are loaded into a memory of the computer.

14. A computer-readable medium (MEM) on which a convolutional neural network (CNN) and program elements are stored that can be read and executed by a computer in order to perform steps of the method for verifying a visual classification architecture of the convolutional neural network (CNN) according to one of the preceding method claims, when the program elements are executed by the computer.

FIG 1

10

14

18

12

16

20

FIG 2

CNN

38

22   24   26   28   30   32   34   36

tc

tc

## FIG 3

CNN

52
Input

54
Filters

56
Convolution

58

60
Max Pooling

Relu

62
Output+VS

## FIG 4

IE

CNN          P          MEM

12

36

VS

V

OE

## FIG 5

## FIG 6

EP 3 654 248 A1

FIG 7

FIG 8

Europäisches
Patentamt

European
Patent Office

Office européen
des brevets

## EUROPEAN SEARCH REPORT

Application Number

EP 18 20 6946

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/344884 A1 (LIN ZHE [US] ET AL) 30 November 2017 (2017-11-30) <br> * paragraph [0020] - paragraph [0027] * <br> * paragraph [0041] - paragraph [0079] * <br> * figures 1-13 * | 1-14 | INV. <br> G06N3/04 <br> G06N3/08 |
| A | CN 108 664 967 A (UNIV SHANGHAI JIAOTONG) 16 October 2018 (2018-10-16) <br> * abstract * | 1-3,7, 11-14 | |
| A,D | SEBASTIAN BACH ET AL: "On Pixel-Wise Explanations for Non-Linear Classifier Decisions by Layer-Wise Relevance Propagation", <br> PLOS ONE, <br> vol. 10, no. 7, <br> 1 January 2015 (2015-01-01), page e0130140, XP055228790, <br> ISSN: 1932-6203, DOI: <br> 10.1371/journal.pone.0130140 <br> * page 1 - page 7 * <br> * page 19 - page 24 * | 1-14 | TECHNICAL FIELDS SEARCHED (IPC) <br><br> G06N |

The present search report has been drawn up for all claims

| Place of search | Date of completion of the search | Examiner |
|---|---|---|
| The Hague | 20 May 2019 | Simigliani, V |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons
 
& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 18 20 6946

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2019

| Patent document cited in search report | | Publication date | Patent family member(s) | Publication date |
|---|---|---|---|---|
| US 2017344884 | A1 | 30-11-2017 | NONE | |
| CN 108664967 | A | 16-10-2018 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BACH, S. ; BINDER, A. ; MONTAVON, G. ; KLAUSCHEN, F. ; MULLER, K.R. ; SAMEK, W.** On pixel-wise explanations for non-linear classifier decisions by layer-wise relevance propagation. *PloS one,* 2015, vol. 10, e0130140
- Top-down neural attention by excitation backprop. **ZHANG, J. ; LIN, Z. ; BRANDT, J. ; SHEN, X. ; SCLAROFF, S.** European Conference on Computer Vision. Springer, 2016, 543-559
- **CAO, C. ; LIU, X. ; YANG, Y. ; YU, Y. ; WANG, J. ; WANG, Z. ; HUANG, Y. ; WANG, L. ; HUANG, C. ; XU, W. et al.** Look and think twice: Capturing top-down visual attention with feedback convolutional neural networks. *Proceedings of the IEEE International Conference on Computer Vision,* 2015, 2956-2964

- **SIMONYAN ; KAREN ; ZISSERMAN ; ANDREW.** Very Deep Convolutional Networks for Large-Scale Image Recognition. *CoRR,* 2014
- **SZEGEDY ; CHRISTIAN et al.** Going deeper with convolutions. *IEEE Conference on Computer Vision and Pattern Recognition (CVPR) (2015),* 2015, 1-9
- **HE, K. ; ZHANG, X. ; REN, S. ; SUN, J.** Deep residual learning for image recognition. *Proceedings of the IEEE conference on computer vision and pattern recognition,* 2016, 770-778
- **SIMONYAN, K. ; ZISSERMAN, A.** Very deep convolutional networks for large-scale image recognition. *arXiv preprint arXiv:1409.1556,* 2014